# EUROPEAN PATENT APPLICATION

(11) **EP 2 023 303 A1**
(43) Date of publication of application: **11.02.2009**
(21) Application number: 08012692.3
(22) Date of filing: 14.07.2008
(51) Int. Cl.: G07F 11/00, A61J 7/00, G06F 19/00

(54) **Apparatus and method for migrating information of patients waiting for drugs to be dispensed**

(30) Priority: 27.07.2007 KR 20070075851
(71) Applicant: JVM Co., Ltd., Daegu 704-170 (KR)
(72) Inventor: Kim, Jun-Ho, Suseong-gu, Daegu (KR)
(74) Representative: Grünecker, Kinkeldey, Stockmair & Schwanhäusser Anwaltssozietät

(57) **Abstract**

An apparatus and method for migrating information of patients waiting for drugs to be dispensed is provided. The apparatus includes Automatic Tablet Dispensing and Packaging Systems (ATDPSs) and a dispensing control server. When dispensing information is received, each ATDPS stores it and performs dispensing. The ATDPS generates dispensing-disabled indication information when an operator has issued a waiting patient migration request or when an error is detected or the number of waiting patients is excessive, and transmits the dispensing-disabled indication information to the server and migrates information of waiting patients, for whom dispensing has been canceled, to the server when a dispensing information migration command is received from the server. When dispensing information of a different ATDPS is migrated to the ATDPS, the ATDPS stores it and performs dispensing. The server performs overall control of dispensing of the ATDPSs. When dispensing-disabled indication information or information requesting distribution of waiting patients is received from a specific ATDPS, the server selects an ATDPS capable of performing dispensing and automatically transmits information of dispensing-canceled patients of the specific ATDPS to the selected ATDPS to control dispensing operations.

## Description

### BACKGROUND OF THE INVENTION

### Field of the Invention

The present invention relates to an apparatus and method for migrating information of patients waiting for drugs to be dispensed, wherein, when a plurality of automatic drug packaging devices (specifically, Automatic Tablet Dispensing and Packaging Systems (ATDPSs)) is controlled in an integrated manner, dispensing information of an automatic drug packaging device that is performing dispensing is automatically transmitted to another automatic drug packaging device as needed to increase the efficiency of dispensing tasks.

### Description of the Related Art

An Automatic Tablet Dispensing and Packaging System (ATDPS) generally includes various types of drug cassettes that contain various types of drugs (specifically, tablets), respectively, and automatically dispenses and packages, when a prescription is input, drugs of each dose from the drug cassettes according to the prescription. More specifically, in response to a signal that a CPU produces and outputs according to the prescription, drugs of each dose are discharged from the drug cassettes to a hopper through a discharge passage and are then discharged through the bottom of the hopper. The discharged drugs of each dose are then packaged in a package on which a dose method has been printed by a printing unit.

Recently, as hospitals or drugstores increase in size, they employ an integrated control system that includes a plurality of ATDPSs that is controlled in an integrated manner using a server.

When dispensing is not possible since an error has occurred or the number of patients waiting for drugs to be dispensed is too great in an ATDPS, the integrated ATDPS control system stops an automatic drug packaging function and generates an alarm or the like to indicate the error state.

Upon recognizing the state of the ATDPS, the manager checks dispensing information of patients waiting for drugs to be dispensed, for whom dispensing has not yet been performed, and manually inputs the dispensing information to another ATDPS so as to perform dispensing based on the dispensing information. Alternatively, after the ATDPS recovers from the error, the manager manually inputs the dispensing information to the ATDPS and issues a dispensing command causing the ATDPS to resume dispensing.

According to the conventional method described above, when an error has occurred in a specific ATDPS or the number of patients waiting for drugs to be dispensed is too many for a specific ATDPS, the manager recognizes the state of the specific ATDPS and manually inputs dispensing information of patients waiting for drugs to be dispensed, for whom dispensing has been canceled due to the error, to another ATDPS or alternatively manually inputs the dispensing information to the specific ATDPS when it recovers from the error to perform dispensing of drugs for the patients awaiting the drugs as described above. This causes the inconvenience of having to manually input dispensing information and also increases the dispensing wait time, causing great inconvenience to patients.

### SUMMARY OF THE INVENTION

Therefore, the present invention has been made in view of the above problems, and it is an object of the present invention to provide an apparatus and method for migrating information of patients waiting for drugs to be dispensed, wherein, in the case where a plurality of automatic drug packaging devices (specifically, Automatic Tablet Dispensing and Packaging Systems (ATDPSs)) is controlled in an integrated manner, dispensing information of a specific automatic drug packaging device that is performing dispensing is automatically transmitted to another automatic drug packaging device when the operator has issued a request to migrate information of patients waiting for drugs to be dispensed of the specific automatic drug packaging device through a manual operation or when an error has occurred or the number of patients waiting for drugs to be dispensed is excessive in the specific automatic drug packaging device, thereby increasing the efficiency of dispensing tasks.

It is another object of the present invention to provide an apparatus and method for migrating information of patients waiting for drugs to be dispensed, wherein, when the number of patients waiting for drugs to be dispensed is great or an error occurs in an automatic drug packaging device or when the operator has issued a patient information migration request, information of patients waiting for drugs to be dispensed can be quickly transmitted from the automatic drug packaging device to another automatic drug packaging device, thereby maximizing user convenience and minimizing loss of dispensing tasks.

In accordance with one aspect of the present invention, the above and other objects can be accomplished' by the provision of an apparatus for migrating information of patients waiting for drugs to be dispensed, the apparatus including a plurality of automatic drug packaging devices, and a dispensing control server, wherein each of the plurality of automatic drug packaging devices stores, when dispensing information is received, the dispensing information and then performs dispensing, each of the plurality of automatic drug packaging devices generates dispensing-disabled indication information when an operator has issued a request to migrate information of patients waiting for drugs to be dispensed through a manual operation or when an error is detected through self-diagnosis or the number of patients waiting for drugs to be dispensed is excessive, and transmits the generated dispensing-disabled indication information to the dispensing control server, and then migrates information of patients waiting for drugs to be dispensed, for whom dispensing has been canceled, to the dispensing control server when a command to migrate dispensing information has been received from the dispensing control server, and each of the plurality of automatic drug packaging devices stores, when dispensing information of a different automatic drug packaging device has been migrated to the automatic drug packaging device, the dispensing information of the different automatic drug packaging device and then performs dispensing according to a dispensing command, and wherein the dispensing control server performs overall control of dispensing of the plurality of automatic drug packaging devices, and the dispensing control server selects an automatic drug packaging device capable of performing dispensing, when dispensing-disabled indication information or information requesting distribution of patients waiting for drugs to be dispensed has been received from a specific automatic drug packaging device, and automatically transmits information of patients waiting for drugs to be dispensed, for whom dispensing has been canceled, of the specific automatic drug packaging device to the selected automatic drug packaging device to control dispensing operations.

In accordance with another aspect of the present invention, there is provided a method for migrating information of patients waiting for drugs to be dispensed of a specific automatic drug packaging device to another automatic drug packaging device in an apparatus for migrating information of patients waiting for drugs to be dispensed, the apparatus including a plurality of automatic drug packaging devices, each storing dispensing information and performing dispensing according to a dispensing command, and a dispensing control server for performing overall control of the plurality of automatic drug packaging devices, the method including the dispensing control server checking whether or not dispensing-disabled indication information has been transmitted from an automatic drug packaging device while controlling the plurality of automatic drug packaging devices, transmitting, when the checked result is that dispensing-disabled indication information has been transmitted from a specific automatic drug packaging device, a command to migrate information of patients waiting for drugs to be dispensed to the specific automatic drug packaging device, receiving, when dispensing information of patients waiting for drugs to be dispensed, for whom dispensing has been canceled, has been transmitted from the specific automatic drug packaging device, the dispensing information of the dispensing-canceled patients and storing the received dispensing information in a database, checking states of a plurality of automatic drug packaging devices other than the specific automatic drug packaging device and selecting an automatic drug packaging device to which the dispensing information of the dispensing-canceled patients can be migrated, and transmitting the dispensing information of the dispensing-canceled patients stored in the database to the selected automatic drug packaging device so that the information of the patients waiting for drugs to be dispensed of the specific automatic drug packaging device is migrated to the selected automatic drug packaging device.

In accordance with another aspect of the present invention, there is provided a method for migrating information of patients waiting for drugs to be dispensed of a specific automatic drug packaging device to another automatic drug packaging device in an apparatus for migrating information of patients waiting for drugs to be dispensed, the apparatus including a plurality of automatic drug packaging devices, each storing dispensing information and performing dispensing according to a dispensing command, and a dispensing control server for performing overall control of the plurality of automatic drug packaging devices, the method including each of the plurality of automatic drug packaging devices storing, when dispensing information is received from the dispensing control server, the received dispensing information in a memory and performing dispensing, storing, when information of patients waiting for drugs to be dispensed of another automatic drug packaging device and a patient information migration command are received from the dispensing control server, the received information of the patients waiting for drugs to be dispensed in the memory and performing dispensing, generating dispensing-disabled indication data when an error has occurred while performing the dispensing, when the number of patients waiting for drugs to be dispensed is excessive, or when an operator has issued a request to migrate patient information through a manual operation and transmitting the generated dispensing-disabled indication data to the dispensing control server, and transmitting, when a command to migrate information of patients waiting for drugs to be dispensed, for whom dispensing has been canceled, is received from the dispensing control server, the information of the dispensing-canceled patients to the dispensing control server so that the information of the dispensing-canceled patients is migrated to another automatic drug packaging device.

According to the present invention, it is possible to increase the efficiency of dispensing tasks, to maximize user convenience, and to minimize loss of dispensing tasks since, in the case where a plurality of Automatic Tablet Dispensing and Packaging Systems (ATDPSs) is controlled in an integrated manner, dispensing information of a specific automatic drug packaging device that is performing dispensing is automatically transmitted to another automatic drug packaging device when an error has occurred or the number of patients waiting for drugs to be dispensed is excessive in the specific automatic drug packaging device or when the operator (pharmacist) has issued a request to migrate information of patients waiting for drugs to be dispensed of the specific automatic drug packaging device through a manual operation.

### BRIEF DESCRIPTION OF THE DRAWINGS

The above and other objects, features and other advantages of the present invention will be more clearly understood from the following detailed description taken in conjunction with the accompanying drawings, in which:
FIG. 1 illustrates a schematic configuration of an apparatus for migrating information of patients waiting for drugs to be dispensed according to the present invention;
FIG. 2 is a block diagram illustrating a configuration of an embodiment of one of a plurality of automatic drug packaging devices in the apparatus for migrating information of patients waiting for drugs to be dispensed according to the present invention;
FIG. 3 is a block diagram illustrating a configuration of an embodiment of a dispensing control server in the apparatus for migrating information of patients waiting for drugs to be dispensed according to the present invention;
FIGS. 4A to 4C illustrate example screens displayed as information of patients waiting for drugs to be dispensed is migrated in the present invention;
FIG. 5 is a flow chart illustrating a method for a dispensing control server migrating information of patients waiting for drugs to be dispensed according to the present invention; and
FIG. 6 is a flow chart illustrating a method for an automatic drug packaging device migrating information of patients waiting for drugs to be dispensed according to the present invention.

### DETAILED DESCRIPTION OF THE PREFERRED EMBODIMENTS

Now, preferred embodiments of the present invention will be described in detail with reference to the accompanying drawings. In the following description of the present invention, a detailed description of known functions and configurations incorporated herein will be omitted when it may obscure the subject matter of the present invention.

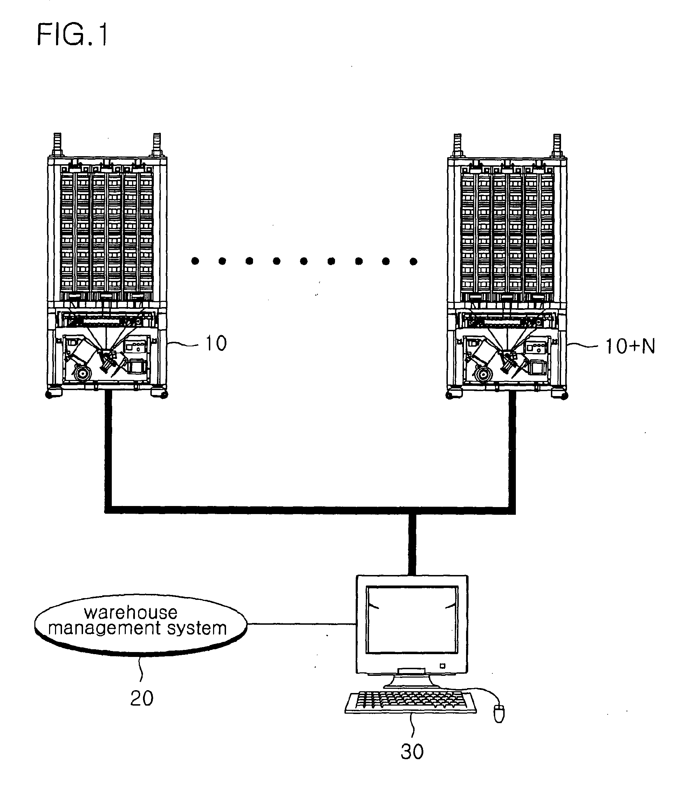

FIG. 1 illustrates a schematic configuration of an apparatus for migrating information of patients waiting for drugs to be dispensed according to the present invention, which includes automatic drug packaging devices (specifically, ATDPSs) 10 to 10+N, a warehouse management system 20, and a dispensing control server 30.

The automatic drug packaging devices 10 to 10+N that automatically package drugs are connected to the dispensing control server 30, which manages the inventory of drugs of the automatic drug packaging devices 10 to 10+N in an integrated manner and checks whether or not each of the devices has malfunctioned, through Local Area Network (LAN) or Controller Area Network (CAN) communication. The dispensing control server 30 is also connected to the warehouse management system 20, which manages drugs and consumables stored in a warehouse through LAN or CAN communication.

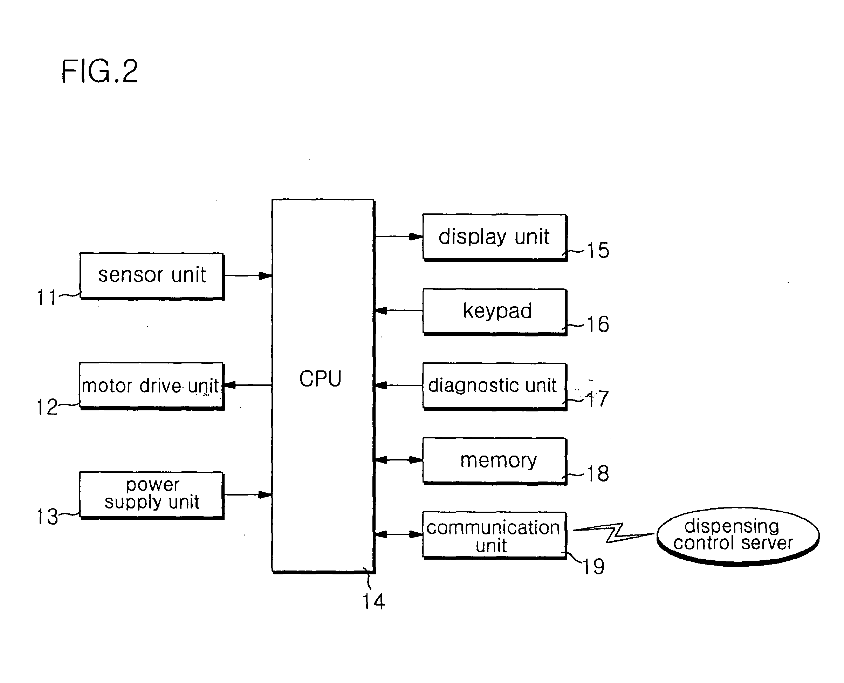

FIG. 2 is a block diagram illustrating the configuration of an automatic drug packaging device 10 among the automatic drug packaging devices 10 to 10+N, each of which has the same configuration and performs the same operations.

As shown, the automatic drug packaging device (specifically, ATDPS) 10 includes a sensor unit 11, a motor drive unit 12, a power supply unit 13, a Central Processing Unit (CPU) 14, a display unit 15, a keypad 16, a self-diagnostic unit 17, a memory 18, and a communication unit 19.

The sensor unit 11 detects loading of drugs, supplying of drugs, presence or absence of drugs, or the like and can be implemented using a variety of sensors such as optical or ultrasonic sensors.

The motor drive unit 12 drives a motor for conveying drugs. The power supply unit 13 supplies required drive power to the motor and each block of the automatic drug packaging device 10.

The display unit 15 displays operating states of the automatic drug packaging device 10 and preferably uses an LCD. The keypad 16 is a key input unit that allows an operator (i.e., pharmacist) to control the operations or functions of the automatic drug packaging device 10 or to input required information to the automatic drug packaging device 10 by manually operating keys.

The memory 18 is a storage device in which a program for controlling operations of the automatic drug packaging device 10 is installed and data generated during control of the automatic drug packaging device 10 is stored. The diagnostic unit 17 detects an error occurring while the automatic drug packaging device 10 is in operation or detects whether or not the number of patients waiting for drugs to be dispensed is excessive.

The communication unit 19 receives dispensing information of the automatic drug packaging device 10 and dispensing information of another automatic drug packaging device from the dispensing control server 30 and transfers the received dispensing information to the CPU 14. The communication unit 19 also transmits dispensing-disabled indication data generated by the CPU 14 to the dispensing control server 30 and transmits dispensing information of patients waiting for drugs to be dispensed, for whom dispensing has been canceled due to the dispensing-disabled indication data generated by the CPU 14, to the dispensing control server 30.

The CPU 14 controls the overall operation of the automatic drug packaging device 10. Specifically, the CPU 14 receives dispensing information of the automatic drug packaging device 10 and dispensing information of another automatic drug packaging device from the communication unit 19 and stores the received dispensing information in the memory 18 and controls the motor drive unit 12 to control a dispensing operation according to the dispensing information. When the diagnostic unit 17 has detected a malfunction, when the number of patients waiting for drugs to be dispensed is excessive, or when the operator has issued a request to migrate information of patients waiting for drugs to be dispensed through the keypad 16, the CPU 14 generates and transmits dispensing-disabled indication data to the dispensing control server 30 through the communication unit 19 and transmits dispensing information of patients waiting for drugs to be dispensed, for whom dispensing has been canceled due to the dispensing-disabled indication data, to the dispensing control server 30 through the communication unit 19.

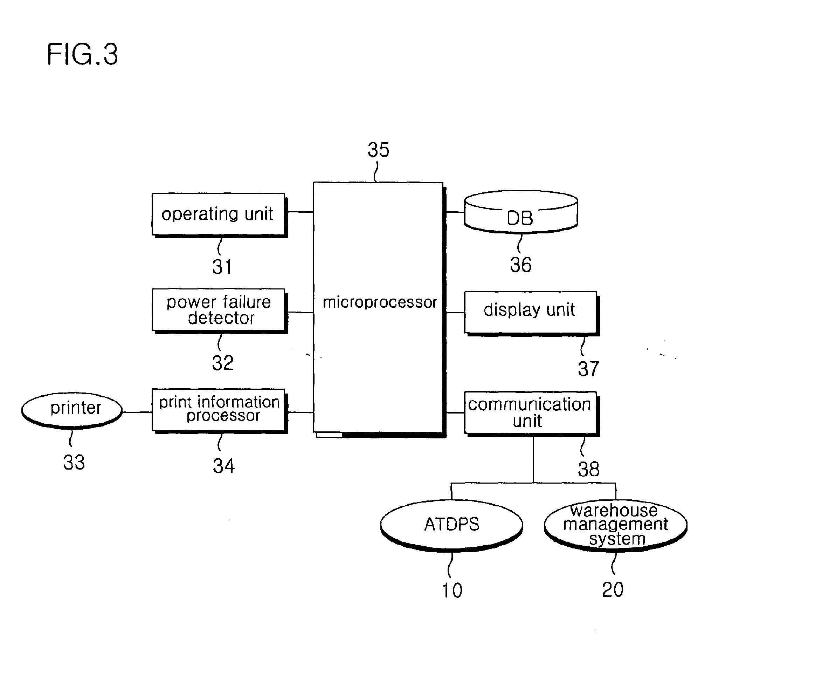

FIG. 3 is a block diagram illustrating a configuration of an embodiment of the dispensing control server in the apparatus for migrating information of patients waiting for drugs to be dispensed according to the present invention. As shown, the dispensing control server 30 includes an operating unit 31, a power failure detector 32, a printer 33, a print information processor 34, a microprocessor 35, a database 36, a display unit 37, and a communication unit 38.

The operating unit 31 is used to input an operating signal for performing a function or to input an operating signal for specifying an automatic drug packaging device for status checking or checking details of the specified automatic drug packaging device. The power failure detector 32 detects power failure of the apparatus.

The print information processor 34 processes print information for outputting information such as drug inventory and information of drugs stored in drug cassettes in each automatic drug packaging device at each unit time and transmits the processed print information to the printer 33.

The display unit 37 displays respective states of the automatic drug packaging devices 10 to 10+N and displays, when information of patients waiting for drugs to be dispensed is migrated, states of the migration of the information of the patients. The display unit 37 is preferably implemented using a Liquid Crystal Display (LCD).

The database 36 is a storage device in which a program for controlling each automatic drug packaging device is installed and dispensing information of each automatic drug packaging device is stored and dispensing information of patients waiting for drugs to be dispensed, for whom dispensing has been canceled in an automatic drug packaging device since an error has occurred in the automatic drug packaging device, is also stored.

The communication unit 38 transmits and receives data to and from the plurality of automatic drug packaging devices 10 to 10+N that automatically dispense and package drugs through communication (preferably, LAN, CAN, RS422, or RS485 communication) with the automatic drug packaging devices 10 to 10+N. The communication unit 38 can also be connected to a wireless Internet to wirelessly transmit a malfunction indication message when a malfunction occurs in the automatic drug packaging device 10 and to wirelessly transmit monitoring information of the current state of the automatic drug packaging device 10 to a mobile phone of the user (i.e., dispensing manager). In addition, the communication unit 38 can transmit and receive data to and from the warehouse management system 20 through internal communication with the warehouse management system 20 that manages the warehouse in which drugs and consumables are stored. The communication unit 38 operates in conjunction with the microprocessor 35 to transmit and receive dispensing information of each of the plurality of automatic drug packaging devices 10 to 10+N or to transmit and receive or to migrate dispensing information of patients waiting for drugs to be dispensed, for whom dispensing has been canceled in an automatic drug packaging device in which an error has occurred.

The microprocessor 35 performs dispensing control of each automatic drug packaging device through the communication unit 38 and acquires inventory and status information of each automatic drug packaging device through the communication unit 38. When an error has occurred in an automatic drug packaging device, the microprocessor 35 performs a control operation to migrate dispensing information of patients waiting for drugs to be dispensed, for whom dispensing has been canceled due to the error in the automatic drug packaging device, to another automatic drug packaging device. The microprocessor 35 also functions to select, when an error has occurred in an automatic drug packaging device, another automatic drug packaging device to which dispensing information of patients waiting for drugs to be dispensed, for whom dispensing has been canceled due to the error in the automatic drug packaging device, is to be migrated.

In the apparatus for migrating information of patients waiting for drugs to be dispensed according to the present invention constructed as described above, while each of the automatic drug packaging devices 10 to 10+N is connected to the dispensing control server 30 through the communication unit 38, the microprocessor 35 of the dispensing control server 30 acquires dispensing information to be transmitted to each automatic drug packaging device in cooperation with the database 36 and transmits the extracted dispensing information to each automatic drug packaging device corresponding to the extracted dispensing information through the communication unit 38.

In this manner, the dispensing control server 30 controls all automatic drug packaging devices connected to the dispensing control server 30 in an integrated manner.

When dispensing information used for dispensing in the automatic drug packaging device 10 is transmitted to the automatic drug packaging device 10 through the communication unit 19, the CPU 14 of the automatic drug packaging device 10 stores the dispensing information in the memory 18 and performs dispensing in cooperation with the motor drive unit 12, the sensor unit 11, etc. A detailed description of basic functions of the automatic drug packaging device 10 such as an automatic drug packaging function is omitted herein since the basic functions of the automatic drug packaging device 10 are similar to those of known technologies and are not directly associated with the present invention.

During dispensing, the diagnostic unit 17 detects whether or not a malfunction (or error) has occurred in the automatic drug packaging device 10 through diagnosis of the automatic drug packaging device 10 and checks whether or not the number of patients waiting for drugs to be dispensed is excessive through analysis of dispensing information in the memory 18. The detection of whether or not a malfunction (or error) has occurred can be easily implemented, for example, using a method of performing self-diagnosis at regular intervals or a method of generating an interrupt when a specific part of the automatic drug packaging device 10 has malfunctioned. The checking of whether or not the number of patients waiting for drugs to be dispensed is excessive can be performed by presetting the number of patients waiting for drugs to be dispensed of the automatic drug packaging device 10 and comparing the number of patients waiting for drugs to be dispensed stored in the memory 18 with the preset number. When an error has occurred so that dispensing is not possible or when the number of patients waiting for drugs to be dispensed is excessive, the diagnostic unit 17 notifies the CPU 14 that dispensing is disabled.

In addition, when a trivial error (for example, a print ribbon refill-related error or a cassette-related error) has occurred during dispensing or when the operator (i.e., pharmacist) desires to transmit information of patients waiting for drugs to be dispensed to another device to achieve efficient distribution of dispensing tasks, the operator selects a function to disable dispensing through the keypad 16 to notify the CPU 14 that dispensing is disabled.

The CPU 14 generates dispensing-disabled indication data based on the notified dispensing-disabled indication information and transmits the dispensing-disabled indication data to the dispensing control server 30 through the communication unit 19 and stops dispensing. When information indicating that dispensing is disabled can be displayed on the display unit 15, it is preferable that corresponding error states be displayed on the display unit 15. It is also preferable that the automatic drug packaging device 10 be implemented so as to enable data communication even when the automatic drug packaging device 10 is in the dispensing-disabled state.

The communication unit 38 of the dispensing control server 30 receives the dispensing-disabled indication data from the automatic drug packaging device 10 and transfers the dispensing-disabled indication data to the microprocessor 35. The microprocessor 35 stores the received dispensing-disabled indication data in the database 36 and also displays the dispensing-disabled indication data on the display unit 37 to enable the operator of the apparatus to be aware of the current state.

The microprocessor 35 then commands, through the communication unit 38, the automatic drug packaging device 10 in which an error has occurred to cancel a list of patients waiting for drugs to be dispensed and to transmit dispensing information of the dispensing-canceled patients.

Upon receiving this command, the CPU 14 of the automatic drug packaging device 10 retrieves dispensing information of the patients waiting for drugs to be dispensed, for whom dispensing has been canceled due to the error in the automatic drug packaging device, among dispensing information of all patients waiting for drugs to be dispensed in cooperation with the memory 18 and transmits the dispensing information of the dispensing-canceled patients to the dispensing control server 30 through the communication unit 19. Here, it is preferable that the CPU 14 separately manage dispensing information, for which dispensing has been performed, and dispensing information, for which dispensing has not been performed, while performing dispensing according to a list of dispensing tasks in cooperation with the memory 18.

Upon receiving the dispensing information of the dispensing-canceled patients, the database 36 of the dispensing control server 30 stores the received dispensing information of the dispensing-canceled patients in the database 36 and analyzes current states of all automatic drug packaging devices, other than the automatic drug packaging device 10 in which the error has occurred, connected to the dispensing control server 30 to select an automatic drug packaging device to which the dispensing information of the dispensing-canceled patients is to be migrated.

Since each automatic drug packaging device transmits its current dispensing state in real time to the dispensing control server 30 through communication therewith, the dispensing control server 30 can detect the state of each automatic drug packaging device in real time.

The microprocessor 35 selects the most appropriate automatic drug packaging device by analyzing the state of each automatic drug packaging device received from the automatic drug packaging device. Here, basically, automatic drug packaging devices that package the same drugs as those of the automatic drug packaging device 10 in which the error has occurred are selected as appropriate automatic drug packaging devices and, preferably, an automatic drug packaging device to which the smallest load is applied among such appropriate automatic drug packaging devices is selected as the most appropriate automatic drug packaging device.

When the most appropriate automatic drug packaging device has been selected in this manner, the microprocessor 35 retrieves the dispensing information of the patients waiting for drugs to be dispensed, for whom dispensing has been canceled, of the automatic drug packaging device 10 from the database 36 and transmits the retrieved dispensing information to the selected automatic drug packaging device and sends a new dispensing command to the selected automatic drug packaging device.

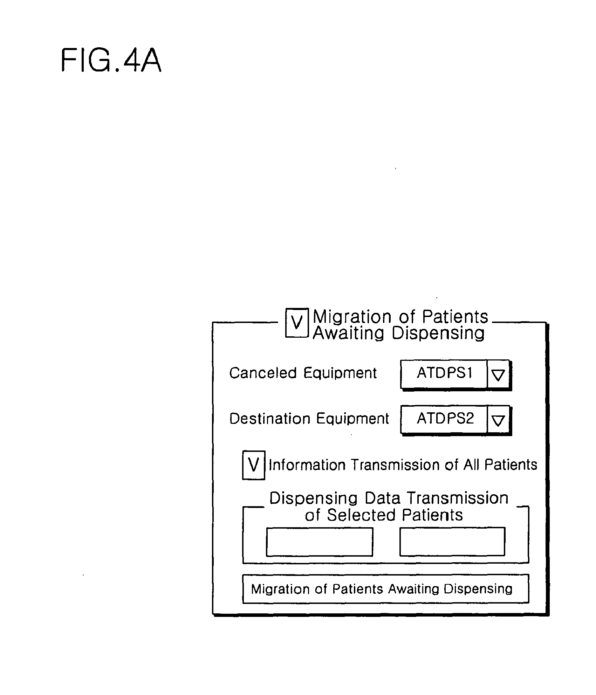

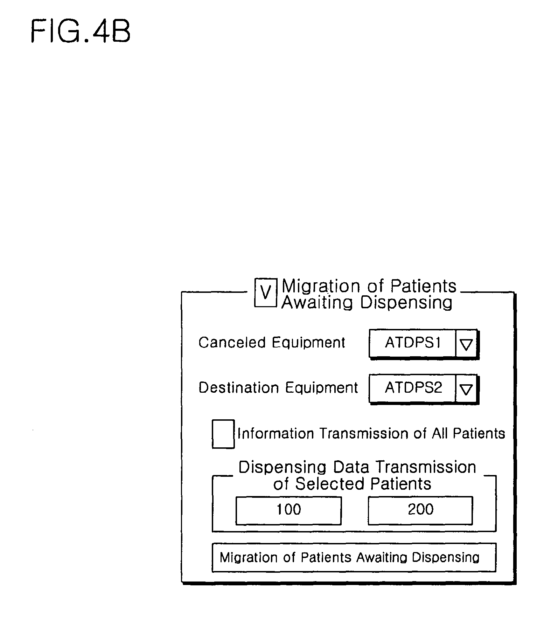

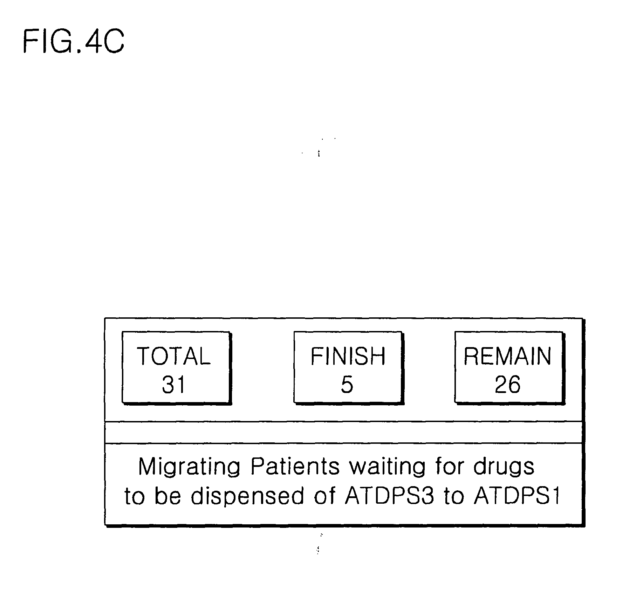

The display unit 37 displays migration states of information of patients waiting for drugs to be dispensed as shown in FIG. 4A to 4C. FIG. 4A illustrates an example screen displayed on the display unit 37 before information of an automatic drug packaging device in which an error has occurred is transmitted to a selected automatic drug packaging device. As shown, this example screen includes a check box indicating whether to select migration of information of patients waiting for drugs to be dispensed, a field for selecting an automatic drug packaging device (ATDPS1) in which dispensing has been canceled due to an error, a field for selecting another (destination) automatic drug packaging device (ATDPS2), a check box indicating whether to select migration of dispensing information of all patients, and a field indicating migration of dispensing information of selected patients. FIG. 4B illustrates an example screen displayed on the display unit 37 after the information of the automatic drug packaging device in which an error has occurred is transmitted to the selected automatic drug packaging device. FIG. 4C illustrates the state of migration of information of patients, for whom dispensing has been canceled, where the total number of dispensing information tasks of patients waiting for drugs to be dispensed is 31, 5 dispensing information tasks of the 31 dispensing information items has been migrated, and the number of remaining dispensing information items is 26.

On the other hand, according to the new dispensing command as described above, the selected automatic drug packaging device stores dispensing information of the automatic drug packaging device, in which an error has occurred, as new dispensing information in a memory of the selected automatic drug packaging, device and performs a normal dispensing function to perform dispensing of the automatic drug packaging device in which an error has occurred.

This eliminates not only the inconvenience of patients having to wait for drugs to be dispensed until the automatic drug packaging device recovers from the error but also the inconvenience of the operator having to manually enter dispensing information of the dispensing-canceled patients to another device as in the related art, thereby maximizing user convenience and minimizing the loss of dispensing tasks (or operations) due to the error.

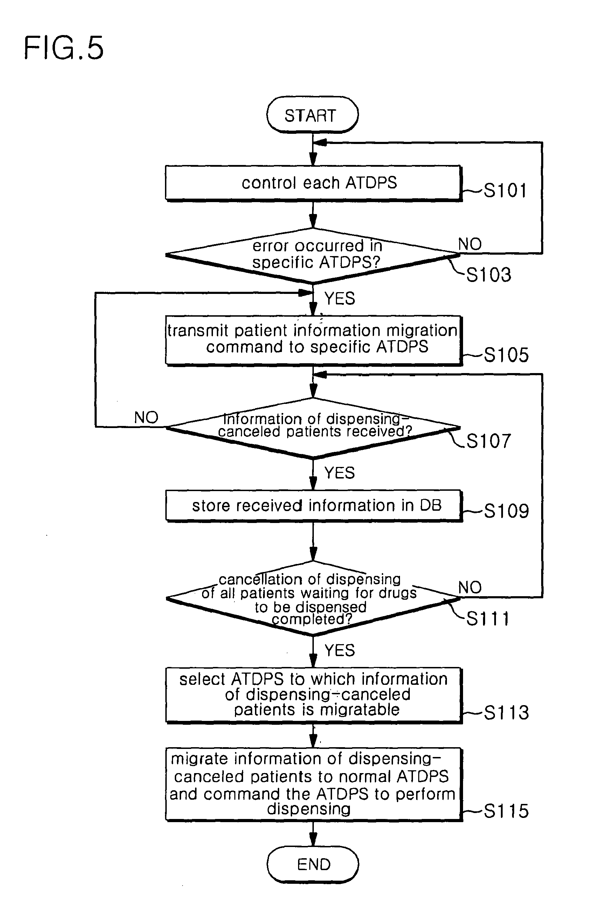

FIG. 5 is a flow chart illustrating a method for a dispensing control server migrating information of patients waiting for drugs to be dispensed according to the present invention, where "S" denotes a step.

As shown in FIG. 5, the method for the dispensing control server migrating information of patients waiting for drugs to be dispensed includes the steps of S101) to S103) checking whether or not dispensing-disabled indication information has been transmitted from an automatic drug packaging device (ATDPS) while controlling a plurality of automatic drug packaging devices, S105) transmitting, when the checked result is that dispensing-disabled indication information has been transmitted from a specific automatic drug packaging device, a command to migrate information of patients waiting for drugs to be dispensed to the specific automatic drug packaging device, S107) to S109) receiving, when dispensing information of patients waiting for drugs to be dispensed, for whom dispensing has been canceled, has been transmitted from the specific automatic drug packaging device, the dispensing information of the dispensing-canceled patients and storing the received dispensing information in a database, S111) to S113) checking states of a plurality of automatic drug packaging devices other than the specific automatic drug packaging device and selecting an automatic drug packaging device to which the dispensing information of the dispensing-canceled patients can be migrated, and S115) transmitting the dispensing information of the dispensing-canceled patients stored in the database to the selected automatic drug packaging device so that the information of the patients waiting for drugs to be dispensed of the specific automatic drug packaging device is migrated to the selected automatic drug packaging device.

The following is a more detailed description of the method for the dispensing control server migrating information of patients waiting for drugs to be dispensed according to the present invention. First, at step S101, the dispensing control server controls a plurality of automatic drug packaging devices connected to the dispensing control server. While controlling the plurality of automatic drug packaging device, the dispensing control server checks whether or not dispensing-disabled indication information has been transmitted from an automatic drug packaging device at step S103. Here, each automatic drug packaging device generates and transmits dispensing-disabled indication data to the dispensing control server when the automatic drug packaging device cannot dispensing since an error has occurred in the automatic drug packaging device while performing dispensing according to a dispensing command, when the number of patients waiting for drugs to be dispensed is excessive in the automatic drug packaging device so that it will take too long to complete dispensing of the patients waiting for drugs to be dispensed, or when an operator has issued a request to migrate patient information in the automatic drug packaging device through a manual operation as needed. Accordingly, the dispensing control server can detect the current states of the automatic drug packaging devices in real time while continuously performing communication with the automatic drug packaging devices that are under control of the dispensing control server.

When the operator has issued a request to migrate patient information through a manual operation, when an error has occurred, or when the number of patients waiting for drugs to be dispensed is excessive in a specific automatic drug packaging device, the dispensing control server proceeds to step S105 to transmit a command to migrate information of patients waiting for drugs to be dispensed to the specific automatic drug packaging device in which an error has occurred.

Upon receiving the command to migrate information patients waiting for drugs to be dispensed, the specific automatic drug packaging device cancels dispensing and transmits dispensing information of patients, for whom dispensing has been canceled, to the dispensing control server through communication therewith.

When it is determined at step S107 that the dispensing information of the dispensing-canceled patients has been transmitted from the specific automatic drug packaging device, the dispensing control server receives and stores the dispensing information of the dispensing-canceled patients in a database at step S109. When it is determined at step S111 that cancellation of dispensing of all patients waiting for drugs to be dispensed has been completed, the dispensing control server proceeds to step S113 to select an automatic drug packaging device to which information of the dispensing-canceled patients is to be migrated.

Here, basically, automatic drug packaging devices that package the same drugs as those of the specific automatic drug packaging device in which an error has occurred are selected as appropriate automatic drug packaging devices and, preferably, an automatic drug packaging device to which the smallest load is applied among such appropriate automatic drug packaging devices is selected as the most appropriate automatic drug packaging device.

Thereafter, at step S115, the dispensing control server 30 transmits the dispensing information of the dispensing-canceled patients stored in the database to the selected automatic drug packaging device so that the information of the patients waiting for drugs to be dispensed of the specific automatic drug packaging device is migrated to the selected automatic drug packaging device. Then, the selected automatic drug packaging device performs dispensing of the dispensing-canceled patients of the specific automatic drug packaging device in which an error has occurred.

This method eliminates not only the inconvenience of patients having to wait for drugs to be dispensed until the automatic drug packaging device recovers from the error but also the inconvenience of the operator having to manually enter dispensing information of the dispensing-canceled patients to another device as in the related art, thereby maximizing user convenience and minimizing the loss of dispensing work (or operations) due to the error.

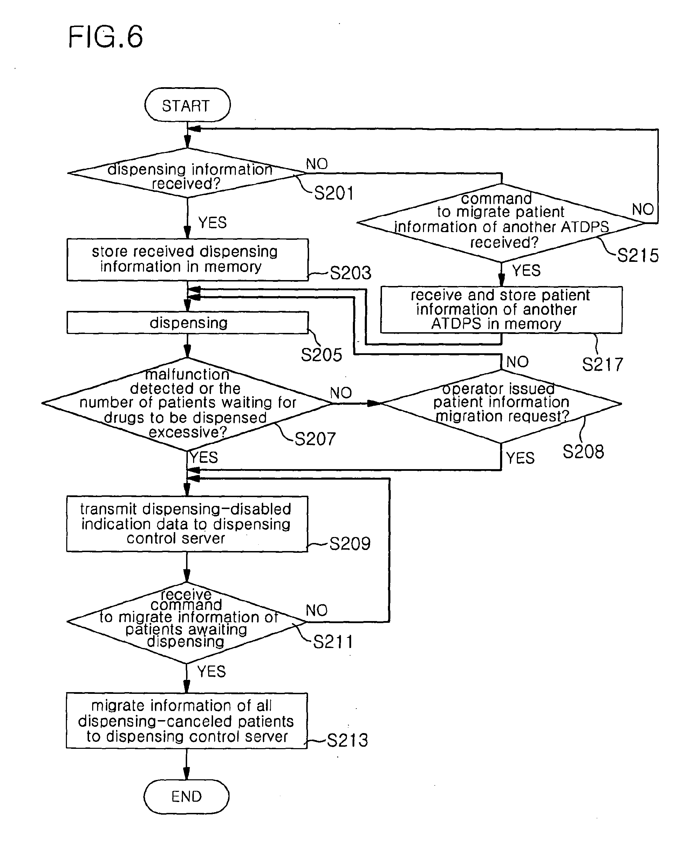

FIG. 6 is a flow chart illustrating a method for an automatic drug packaging device migrating information of patients waiting for drugs to be dispensed according to the present invention, where "S" denotes a step.

As shown in FIG. 6, the method for an automatic drug packaging device migrating information of patients waiting for drugs to be dispensed includes the steps of S201) to S205) storing, when dispensing information is received from the dispensing control server, the received dispensing information in a memory and performing dispensing, S215) to S217) and S205) storing, when information of patients waiting for drugs to be dispensed of another automatic drug packaging device and a patient information migration command are received from the dispensing control server, the received information of the patients waiting for drugs to be dispensed in the memory and performing dispensing, S207) to S209) generating dispensing-disabled indication data when an error has occurred while performing the dispensing, when the number of patients waiting for drugs to be dispensed is excessive, or when an operator has issued a request to migrate patient information through a manual operation and transmitting the generated dispensing-disabled indication data to the dispensing control server, and S211) to S213) transmitting, when a command to migrate information of patients waiting for drugs to be dispensed, for whom dispensing has been canceled, is received from the dispensing control server, the information of the dispensing-canceled patients to the dispensing control server so that the information of the dispensing-canceled patients is migrated to another automatic drug packaging device.

The following is a more detailed description of the method for an automatic drug packaging device migrating information of patients waiting for drugs to be dispensed according to the present invention. First, at step S201, the automatic drug packaging device checks whether or not dispensing information has been received from the dispensing control server. When dispensing information has been received from the dispensing control server, the automatic drug packaging device stores the received dispensing information in a memory at step S203 and then proceeds to step S205 to perform dispensing.

While performing dispensing, the automatic drug packaging device checks, at step S207, whether or not an error has occurred or the number of patients waiting for drugs to be dispensed is excessive and checks, at step S208, whether or not an operator has issued a request to migrate patient information through a manual operation. If the checked result is that an error has occurred, the number of patients waiting for drugs to be dispensed is excessive or the operator has issued a patient information migration request through a manual operation, the automatic drug packaging device proceeds to step S209 to generate and transmits dispensing-disabled indication information to the dispensing control server.

Thereafter, at step S211, the automatic drug packaging device checks whether or not a command to migrate information of patients waiting for drugs to be dispensed has been received. When the checked result is that a command to migrate information of patients waiting for drugs to be dispensed has been received, the automatic drug packaging device proceeds to step S213 to retrieve dispensing information of patients waiting for drugs to be dispensed, for whom dispensing has been canceled, and to transmit the dispensing information of the dispensing-canceled patients to the dispensing control server.

Then, the dispensing control server receives the dispensing information of the automatic drug packaging device in which an error has occurred and selects another automatic drug packaging device that can perform dispensing and then transmits the dispensing information of the automatic drug packaging device in which an error has occurred to the selected automatic drug packaging device, thereby achieving rapid dispensing.

On the other hand, if the result of checking at step S201 is that no dispensing information has been received, the automatic drug packaging device proceeds to step S215 to check whether or not a command to migrate patient information of another automatic drug packaging device has been received. If the checked result is that a command to migrate patient information of a specific automatic drug packaging device has been received, the automatic drug packaging device proceeds to step S217 to receive dispensing information of patients waiting for drugs to be dispensed of the specific automatic drug packaging device through communication with the specific automatic drug packaging device and to store the received dispensing information of the patients waiting for drugs to be dispensed in the memory.

Then, the automatic drug packaging device proceeds to step S205 to repeat the subsequent steps until dispensing is completed.

As is apparent from the above description, the present invention provides an apparatus and method for migrating information of patients waiting for drugs to be dispensed with a variety of advantages. For example, it is possible to increase the efficiency of dispensing tasks, to maximize user convenience, and to minimize loss of dispensing tasks since, in the case where a plurality of automatic drug packaging devices (specifically, Automatic Tablet Dispensing and Packaging Systems (ATDPSs)) is controlled in an integrated manner, dispensing information of a specific automatic drug packaging device that is performing dispensing is automatically transmitted to another automatic drug packaging device when an error has occurred or the number of patients waiting for drugs to be dispensed is excessive in the specific automatic drug packaging device or when the operator manually requests migration of information of patients waiting for drugs to be dispensed of the specific automatic drug packaging device.

In addition, when an error has occurred in an automatic drug packaging device or a patient information migration request has been issued in an automatic drug packaging device, dispensing information of the automatic drug packaging device can be easily transmitted to another automatic drug packaging device. This eliminates the inconvenience of having to manually input information of patients waiting for drugs to be dispensed, for whom dispensing has been canceled due to the error, to the automatic drug packaging device that has recovered from the error, or to another automatic drug packaging device.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An apparatus for migrating information of patients waiting for drugs to be dispensed, the apparatus comprising:
a plurality of automatic drug packaging devices; and
a dispensing control server,
wherein each of the plurality of automatic drug packaging devices stores, when dispensing information is received, the dispensing information and then performs dispensing,
each of the plurality of automatic drug packaging devices generates dispensing-disabled indication information when an operator has issued a request to migrate information of patients waiting for drugs to be dispensed through a manual operation or when an error is detected through self-diagnosis or the number of patients waiting for drugs to be dispensed is excessive, and transmits the generated dispensing-disabled indication information to the dispensing control server, and then migrates information of patients waiting for drugs to be dispensed, for whom dispensing has been canceled, to the dispensing control server when a command to migrate dispensing information has been received from the dispensing control server, and
each of the plurality of automatic drug packaging devices stores, when dispensing information of a different automatic drug packaging device has been migrated to the automatic drug packaging device, the dispensing information of the different automatic drug packaging device and then performs dispensing according to a dispensing command, and
wherein the dispensing control server performs overall control of dispensing of the plurality of automatic drug packaging devices, and
the dispensing control server selects an automatic drug packaging device capable of performing dispensing, when dispensing-disabled indication information or information requesting distribution of patients waiting for drugs to be dispensed has been received from a specific automatic drug packaging device, and automatically transmits information of patients waiting for drugs to be dispensed, for whom dispensing has been canceled, of the specific automatic drug packaging device to the selected automatic drug packaging device to control dispensing operations.

2. The apparatus according to claim 1, wherein each of the plurality of automatic drug packaging devices includes:
a power supply unit for supplying required drive power to a motor and each block of the automatic drug packaging device;
a display unit for displaying operating states of the automatic drug packaging device;
a keypad that is a key input unit allowing a manager or operator to control operations or functions of the automatic drug packaging device or to input required information to the automatic drug packaging device by manually operating keys;
a memory that is a storage device in which a program for controlling operations of the automatic drug packaging device is installed and data generated during control of the automatic drug packaging device is stored;
a diagnostic unit for detecting an error occurring while the automatic drug packaging device is in operation or detecting whether or not the number of patients waiting for drugs to be dispensed is excessive;
a communication unit for receiving dispensing information of the automatic drug packaging device and dispensing information of another automatic drug packaging device from the dispensing control server and transferring the received dispensing information to a Central Processing Unit (CPU) and transmitting dispensing-disabled indication data generated by the CPU to the dispensing control server and transmitting dispensing information of patients waiting for drugs to be dispensed, for whom dispensing has been canceled due to the dispensing-disabled indication data generated by the CPU, to the dispensing control server; and
the CPU for controlling overall operations of the automatic drug packaging device, wherein the CPU receives dispensing information of the automatic drug packaging device and dispensing information of another automatic drug packaging device from the communication unit and stores the received dispensing information in the memory and controls the motor drive unit to control a dispensing operation according to the dispensing information and the CPU generates and transmits, when the diagnostic unit has detected a malfunction or when the number of patients waiting for drugs to be dispensed is excessive, dispensing-disabled indication data to the dispensing control server through the communication unit and transmits dispensing information of patients waiting for drugs to be dispensed, for whom dispensing has been canceled due to the dispensing-disabled indication data, to the dispensing control server through the communication unit.

3. The apparatus according to claim 1, wherein the dispensing control server includes:
an operating unit used to input an operating signal for performing a function or to input an operating signal for specifying an automatic drug packaging device for status checking or checking details of the specified automatic drug packaging device;
a display unit for displaying respective states of the automatic drug packaging devices and displays, when information of patients waiting for drugs to be dispensed is migrated, states of the migration of the information of the patients;
a database that is a data storage device in which a program for controlling each automatic drug packaging device is installed and dispensing information of each automatic drug packaging device is stored and dispensing information of patients waiting for drugs to be dispensed, for whom dispensing has been canceled in an automatic drug packaging device since an error has occurred in the automatic drug packaging device, is also stored;
a communication unit for transmitting and receiving dispensing information of an automatic drug packaging device or for transmitting and receiving or for migrating dispensing information of patients waiting for drugs to be dispensed, for whom dispensing has been canceled in an automatic drug packaging device in which an error has occurred, through communication with the plurality of automatic drug packaging devices that automatically dispense and package drugs; and
a microprocessor for performing dispensing control of each automatic drug packaging device through the communication unit, acquiring inventory and status information of each automatic drug packaging device through the communication unit, and performing, when an error has occurred in an automatic drug packaging device, a control operation to migrate dispensing information of patients waiting for drugs to be dispensed, for whom dispensing has been canceled due to the error in the automatic drug packaging device, to another automatic drug packaging device.

4. The apparatus according to claim 3, wherein the microprocessor also functions to select, when an error has occurred in an automatic drug packaging device, another automatic drug packaging device to which dispensing information of patients waiting for drugs to be dispensed, for whom dispensing has been canceled due to the error in the automatic drug packaging device, is to be migrated.

5. A method for migrating information of patients waiting for drugs to be dispensed of a specific automatic drug packaging device to another automatic drug packaging device in an apparatus for migrating information of patients waiting for drugs to be dispensed, the apparatus including a plurality of automatic drug packaging devices, each storing dispensing information and performing dispensing according to a dispensing command, and a dispensing control server for performing overall control of the plurality of automatic drug packaging devices, the method comprising:
the dispensing control server checking whether or not dispensing-disabled indication information has been transmitted from an automatic drug packaging device while controlling the plurality of automatic drug packaging devices;
transmitting, when the checked result is that dispensing-disabled indication information has been transmitted from a specific automatic drug packaging device, a command to migrate information of patients waiting for drugs to be dispensed to the specific automatic drug packaging device;
receiving, when dispensing information of patients waiting for drugs to be dispensed, for whom dispensing has been canceled, has been transmitted from the specific automatic drug packaging device, the dispensing information of the dispensing-canceled patients and storing the received dispensing information in a database;
checking states of a plurality of automatic drug packaging devices other than the specific automatic drug packaging device and selecting an automatic drug packaging device to which the dispensing information of the dispensing-canceled patients can be migrated; and
transmitting the dispensing information of the dispensing-canceled patients stored in the database to the selected automatic drug packaging device so that the information of the patients waiting for drugs to be dispensed of the specific automatic drug packaging device is migrated to the selected automatic drug packaging device.

6. The method according to claim 5, wherein the dispensing-disabled indication information includes dispensing-disabled indication information generated when an error has occurred that disables dispensing, dispensing-disabled indication information generated when the number of patients waiting for drugs to be dispensed is excessive so that a long time is required to complete dispensing, or dispensing-disabled indication information generated when an operator has issued a request to migrate patient information through a manual operation.

7. A method for migrating information of patients waiting for drugs to be dispensed of a specific automatic drug packaging device to another automatic drug packaging device in an apparatus for migrating information of patients waiting for drugs to be dispensed, the apparatus including a plurality of automatic drug packaging devices, each storing dispensing information and performing dispensing according to a dispensing command, and a dispensing control server for performing overall control of the plurality of automatic drug packaging devices, the method comprising:
each of the plurality of automatic drug packaging devices storing, when dispensing information is received from the dispensing control server, the received dispensing information in a memory and performing dispensing;
storing, when information of patients waiting for drugs to be dispensed of another automatic drug packaging device and a patient information migration command are received from the dispensing control server, the received information of the patients waiting for drugs to be dispensed in the memory and performing dispensing;
generating dispensing-disabled indication data when an error has occurred while performing the dispensing, when the number of patients waiting for drugs to be dispensed is excessive, or when an operator has issued a request to migrate patient information through a manual operation and transmitting the generated dispensing-disabled indication data to the dispensing control server; and
transmitting, when a command to migrate information of patients waiting for drugs to be dispensed, for whom dispensing has been canceled, is received from the dispensing control server, the information of the dispensing-canceled patients to the dispensing control server so that the information of the dispensing-canceled patients is migrated to another automatic drug packaging device.

8. A method for migrating information of patients waiting for drugs to be dispensed of a specific automatic drug packaging device to another automatic drug packaging device in an apparatus for migrating information of patients waiting for drugs to be dispensed, the apparatus including a plurality of automatic drug packaging devices, each storing dispensing information and performing dispensing according to a dispensing command, and a dispensing control server for performing overall control of the plurality of automatic drug packaging devices, the method comprising:
the dispensing control server checking whether or not dispensing-disabled indication information has been transmitted from an automatic drug packaging device while controlling the plurality of automatic drug packaging devices;
transmitting, when the checked result is that dispensing-disabled indication information has been transmitted from a specific automatic drug packaging device, a command to migrate information of patients waiting for drugs to be dispensed to the specific automatic drug packaging device;
receiving, when dispensing information of patients waiting for drugs to be dispensed, for whom dispensing has been canceled, has been transmitted from the specific automatic drug packaging device, the dispensing information of the dispensing-canceled patients and storing the received dispensing information in a database;
checking states of a plurality of automatic drug packaging devices other than the specific automatic drug packaging device and selecting an automatic drug packaging device to which the dispensing information of the dispensing-canceled patients can be migrated; and
transmitting the dispensing information of the dispensing-canceled patients stored in the database to the selected automatic drug packaging device so that the information of the patients waiting for drugs to be dispensed of the specific automatic drug packaging device is migrated to the selected automatic drug packaging device,
the method further comprising:
each of the plurality of automatic drug packaging devices storing, when dispensing information is received from the dispensing control server, the received dispensing information in a memory and performing dispensing;
storing, when information of patients waiting for drugs to be dispensed of another automatic drug packaging device and a patient information migration command are received from the dispensing control server, the received information of the patients waiting for drugs to be dispensed in the memory and performing dispensing;
generating dispensing-disabled indication data when an error has occurred while performing the dispensing, when the number of patients waiting for drugs to be dispensed is excessive, or when an operator has issued a request to migrate patient information through a manual operation and transmitting the generated dispensing-disabled indication data to the dispensing control server; and
transmitting, when a command to migrate information of patients waiting for drugs to be dispensed, for whom dispensing has been canceled, is received from the dispensing control server, the information of the dispensing-canceled patients to the dispensing control server so that the information of the dispensing-canceled patients is migrated to another automatic drug packaging device,
whereby, when a specific automatic drug packaging device is disabled from performing dispensing, dispensing information of patients waiting for drugs to be dispensed, for whom dispensing has been canceled, of the specific automatic drug packaging device is automatically migrated to a different automatic drug packaging device so that the different automatic drug packaging device performs dispensing of the dispensing-canceled patient.
